# FASCICULE DE BREVET EUROPEEN

(11) **EP 4 185 594 B1**
(45) Date de publication et mention de la délivrance du brevet: **04.12.2024**
(21) Numéro de dépôt: 21739523.5
(22) Date de dépôt: 19.07.2021
(51) Int. Cl.: C07F 11/00, A01N 55/02, A61K 31/555, A01N 59/16

(54) **COMPLEMENT ALIMENTAIRE A BASE DE MOLYBDENE POUR LES ABEILLES**
MOLYBDÄNHALTIGER FUTTERZUSATZ FÜR BIENEN
MOLYBDENUM-BASED FEED SUPPLEMENT FOR BEES

(30) Priorité: 23.07.2020 FR 2007784
(43) Date de publication de la demande: 31.05.2023
(73) Titulaire: Université de Versailles Saint-Quentin-en-Yvelines, 78035 Versailles Cédex (FR); Centre National de la Recherche Scientifique, 75016 Paris (FR); Institutul de Zoologie, Chisinau, 2028 (MD); Universitatea De Stat Din Moldova, Chisinau, 2009 (MD)
(72) Inventeur: FUIOR, Arcadie, 78000 VERSAILLES (FR); FLOQUET, Sébastien, 78000 VERSAILLES (FR); CEBOTARI, Valentina, CHISINAU, 2028 (MD); CEBOTARI, Diana, 78000 VERSAILLES (FR); GULEA, Aurelian, CHISINAU, 2009 (MD); TODERAS, Ion, CHISINAU, 2028 (MD)
(74) Mandataire: IPAZ
(86) Numéro de dépôt international: PCT/EP2021/070097
(87) Numéro de publication internationale: WO 2022/018009

(56) Documents cités:
- DE-A1- 102005 028 170
- JP-A- 2011 116 700
- MD-A2- 20 150 005
- KAST CHRISTINA ET AL: "Distribution of coumaphos in beeswax after treatment of honeybee colonies with CheckMite against the parasitical mite", APIDOLOGIE, ARBEITSGEMEINSCHAFT DER INSTITUTE FUER BIENENFORSCHUNG, CELLE, DE, vol. 51, no. 1, 2 December 2019 (2019-12-02), pages 112 - 122, XP037029092, ISSN: 0044-8435, [retrieved on 20191202], DOI: 10.1007/S13592-019-00724-6

## Description

La présente invention concerne les compléments alimentaires pour les abeilles comprenant des complexes de molybdène et une méthode de prévention contre l'infestation des abeilles et de leurs larves par l'acarien *Varroa destructor.*

La mortalité importante des abeilles, pollinisateurs essentiels à la reproduction des espèces végétales dans le monde, est un problème environnemental et sociétal majeur (Cf. Rapport d'information n°474 du Sénat en date du 22 mars 2017 sur « la lutte contre le déclin des pollinisateurs » et le Plan de développement durable de l'apiculture). Aujourd'hui 20000 espèces végétales sont concernées par la disparition des abeilles et 40% de notre alimentation en dépend directement.

Les filières agricoles et les productions de fruits, de légumes et de semences sont également directement impactées par leur déclin.

Les causes de ce déclin sont multiples (pesticides, frelon asiatique, bactéries, champignons, acariens, ...). Cependant, l'acarien *Varroa destructor* apparu en France en 1982, constitue un problème majeur de l'apiculture mondiale. Ces acariens se développent sur les abeilles et leurs larves causant des malformations, et un affaiblissement des abeilles, plus sensibles alors aux bactéries, champignons (tels que *Nosema Ceranae*) et autres maladies. Ces multiples facteurs ont pour conséquence une mortalité importante des colonies d'abeilles à travers le monde qui peut atteindre jusqu'à 90% par an dans certaines régions du monde. Il est admis que des colonies fortement touchées ne peuvent survivre. Des traitements existent mais ils sont pour le moment à base de produits acaricides, toxiques et probablement cancérigènes pouvant eux-mêmes décimer les colonies et contaminer le miel, la cire et en bout de chaine le consommateur. Ces traitements sont donc exclusivement employés pendant la période hivernale ou hors production de miel.

A ce jour, il existe deux types de produits sur les marchés français et européens : les produits vétérinaires pour le traitement médicamenteux contre *Varroa destructor* et les produits de nourrissement des abeilles comprenant les éléments nutritifs, souvent à base de sucres, protéines, vitamines, et/ou d'extraits de biomasse.

Les produits vétérinaires pour lutter contre *Varroa destructor* comprennent souvent des molécules toxiques, cancérigènes, tératogènes et qui peuvent se retrouver dans le miel et la cire.

Récemment, Ziegelmann et al. (Scientific Reports 2018, 8, DOI: https://doi.org/10.1038/s41598-017-19137-5) décrivent que le chlorure de lithium présente un effet contre *Varroa destructor* par mode d'action systémique lorsque le composé est administré à une concentration entre 2mM et 25mM pendant au moins 24 heures. Les sels de lithium ont été utilisés jusqu'à présent comme substances antidépressives chez l'homme. Son accumulation chez les humains, lorsqu'elle dépasse un certain niveau, peut être toxique. Divers résultats d'études *in vivo* ou *in vitro* montrent que la toxicité du lithium apparait à des lithémies supérieures à 2 mmol/l. L'utilisation du chlorure de lithium à une concentration élevée pourrait engendrer un risque sanitaire tant pour les apiculteurs que pour les consommateurs.

En revanche, les produits de nourrissement des abeilles sont non-toxiques pour les abeilles et les humains car ils comprennent essentiellement des éléments d'origine naturelle, tels que les sucres, les protéines, les vitamines, et/ou les extraits de biomasse. Cependant, l'efficacité de ces produits pour augmenter la production du miel n'est pas scientifiquement prouvée. Ces produits disponibles sur le marché ne montrent aucun effet pour protéger les abeilles et leurs larves contre l'infestation par *Varroa destructor.*

Le molybdène est un oligoélément indispensable à la vie sur Terre, aussi bien dans les plantes que pour les animaux ou l'homme. Il est d'ailleurs naturellement présent dans les abeilles (environ 40 ng/abeille d'après nos études).

Le brevet moldave MD4438 décrit un procédé de nourrissement des abeilles en utilisant le composé [tétraoxo-éthylènediaminotétraacétato-dimolybdate(V)] de bis-(tétraphénylphosphonium) disemihydrate (dénoté PPh₄-[Mo₂O₄]-EDTA), ainsi qu'un procédé pour le produire. Ce composé montre un effet pour stimuler les fonctions d'ovogénèse des reines, augmenter la quantité de larves, améliorer le développement quantitatif des familles d'abeilles et augmenter leur productivité.

L' agent Perizin connu pour son utilisation dans la prévention de l'infestation des abeilles par l'acarien Varroa Destructor est mentionné dans l'article par Christina Kast et al. Apidologie (2020) 51:112-122.

MD 20150005 A2 décrit des complexes de molybdène utilisés pour nourrir les abeilles.

L'élaboration de nouveaux compléments alimentaires pour aider les abeilles à lutter contre ces agressions extérieures permettrait également de faire chuter la mortalité dans les colonies d'abeilles domestiques.

Cette mortalité est particulièrement forte pendant la période hivernale. Cette mortalité hivernale est variable suivant les régions du monde. En Californie cette mortalité peut atteindre un niveau record de 80 % selon les années, ce qui est considérable.

Ainsi, les attentes des apiculteurs restent extrêmement fortes pour de nouveaux produits non toxiques qui permettent :
- de diminuer significativement la mortalité des colonies d'abeilles, en particulier pendant la période hivernale,
- de lutter contre le parasite *Varroa destructor,* tout au long de l'année,
- de favoriser le développement des colonies,
- d'augmenter la production de miel, dans un contexte de diminution mondiale de la production alors que la demande est en augmentation.

Il est donc nécessaire de développer de nouveaux produits non toxiques permettant à la fois de stimuler efficacement la croissance des abeilles et d'augmenter la productivité du miel, de fournir aux abeilles et aux larves une protection contre *Varroa destructor* et enfin de diminuer la mortalité, notamment hivernale, des colonies d'abeilles.

Les Inventeurs ont mis au point une famille de complexes de molybdène de formule I :

A1ₘ-[Mo₂O₂E₂]-Lₙ,

dans laquelle :
- A1 est un cation choisi parmi les cations des métaux alcalins, les cations des métaux alcalino-terreux, les cations des métaux de transition, ou un cation organique PPh₄⁺
- E est choisi parmi O ou S,
- L est un ligand choisi parmi les acides aminés tels que glycine (gly⁻), cystéine (L-cys²⁻) ou histidine (L-his⁻) ou des ligands polycarboxylates tels que oxalate (Ox²⁻), citrate (Cit³⁻), éthylènediaminetetraacétate (EDTA⁴⁻), nitrilo-triacétate (HNTA²⁻), et imino-diacétate (IDA²⁻),
- m est le nombre de cations et est choisi parmi 0, 1, 2, 3 et 4 ;
- n est le nombre de ligands et est choisi parmi 1 et 2.

Les complexes de molybdène de formule I comprennent tous deux atomes de molybdène au degré d'oxydation +V (Mo(V)), qui sont reliés par une liaison Mo-Mo et soit par des atomes de soufres (groupes sulfures pontants S²⁻) soit par des atomes d'oxygène (groupes Oxo pontants O²⁻). Les sphères de coordination autours des atomes de Mo(V) sont respectivement complétées par un ligand oxo terminal pour donner ce qui constitue le coeur de cette famille de complexe : les clusters [Mo₂O₂S₂]²⁺ (dioxo-di-µ-sulfido-dimolybdène(V)) ou [Mo₂O₄]²⁺ (dioxo-di-µ-oxo-dimolybdène(V)). Le cluster est ensuite complété par d'autres ligands de manière à ce que chaque atome de Mo(V) soit penta ou hexacoordinné. Les complexes ainsi complétés sont de charge neutre ou le plus souvent négative. Dans ce dernier cas, ils sont associés aux cations qui peuvent être des cations inorganiques ou un cation organique PPh₄⁺ (tetraphénylphosphonium) pour équilibrer la charge du complexe à une charge nulle.

Contre toute attente, il est observé que le nourrissement des abeilles par un complément alimentaire comprenant une dose annuelle de l'ordre de quelques micromoles d'un complexe de molybdène de formule I permet non seulement d'augmenter la production du miel et de baisser la mortalité hivernale des abeilles, mais surtout de diminuer significativement l'infestation des abeilles et/ou de leurs larves par *Varroa destructor.*

Les complexes de molybdène de formule I permettent de diminuer jusqu'à 62% l'infestation de *Varroa* sur les abeilles d'une colonie et jusqu'à 82% sur les larves d'une colonie.

Il est à noter, qu'à ce jour, il n'existe aucun traitement connu pour protéger les larves des abeilles contre *Varroa.*

Par ailleurs, aucun traitement ou complément alimentaire du marché ne permet aujourd'hui à la fois de protéger les larves des abeilles contre *Varroa,* de favoriser le développement des colonies, de diminuer la mortalité, notamment hivernale, des abeilles et par conséquent d'augmenter la production de miel.

Sans vouloir se lier à une quelconque théorie de l'invention, l'action des produits n'est pas directe sur *Varroa* mais passe par la stimulation probable du système immunitaire des abeilles qui leur permet de mieux lutter contre ce parasite.

Il est aussi surprenant de constater que les complexes de molybdène de formule I, lorsqu'ils ne comportent pas de cation organique, produisent des effets bénéfiques encore plus significatifs par rapport au complexe PPh₄-[Mo₂O₄]-EDTA divulgué antérieurement, sur la quantité de miel produite lors de la première récolte.

Ainsi, le premier aspect de la présente invention concerne un complexe de molybdène de formule I suivante :

A1ₘ-[Mo₂O₂E₂]-Lₙ, I

dans laquelle :
- A1 est un cation choisi parmi les cations des métaux alcalins, les cations des métaux alcalino-terreux, les cations des métaux de transition, ou un cation organique PPh₄⁺
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou des larves par l'acarien *Varroa Destructor.*

Le nombre m du cation et le nombre n du ligand dans un complexe de molybdène de formule I sont déterminés selon la règle suivante : le(s) ligand(s) L dans un complexe de molybdène de formule I permet(tent) de compléter les clusters [Mo₂O₂S₂]²⁺ ou [Mo₂O₄]²⁺ de manière à ce que chaque atome de Mo(V) soit penta ou hexacoordinné et que les cations dans le complexe de molybdène de formule I permettent d'équilibrer la charge électrique du complexe à une charge nulle.

Par conséquent, le nombre de ligands L dans un complexe de molybdène de formule I dépend de la denticité du ligand choisi.

Par exemple, lorsque le ligand L est un ligand hexadenté, tel que l'EDTA, un complexe de molybdène de formule I comprend un seul ligand. En revanche, deux ligands tridentés tels que L-cystéine, la L-histidine, le citrate, le nitrilo-triacétate, ou l'imino-diacétate sont nécessaires pour hexacoordinner les deux atomes de Mo(V) des clusters [Mo₂O₂S₂]²⁺ ou [Mo₂O₄]²⁺. Dans le cas des ligands bidentés tels que la glycine ou l'ion oxalate (Ox²⁻), deux molécules d'eau viennent compléter les sphères de coordination autour des atomes de molybdène en plus de deux ligands dont chacun se trouve dans la sphère de coordination d'un atome de molybdène.

Dans le cadre de la présente invention, on entend par « ligand polycarboxylate » ligand possédant plusieurs fonctions carboxylates. Comme exemple de ligands polycarboxylates, on peut citer l'oxalate (Ox²⁻) ou le citrate (Cit³⁻).

Le nombre de cations dans un complexe de formule I de l'invention dépend à la fois de la charge et du nombre de ligands et de la valence du cation.

Par exemple, le cluster [Mo₂O₂E₂]²⁺complété par la glycine (gly-) ou l'histidine (L-his⁻) comme ligand est de charge neutre. Par conséquent, dans un complexe de molybdène de formule I, lorsque L est gly⁻ ou L-his⁻, ledit complexe ne comporte pas de cation.

Dans le cadre de la présente invention, lorsque la présence de cation est nécessaire pour équilibrer la charge du complexe à une charge nulle, ledit cation peut être choisi parmi les cations des métaux alcalins, les cations des métaux alcalino-terreux, les cations des métaux de transition.

A titre d'exemples des cations de métaux alcalins qui peuvent être utilisés dans le cadre de la présente invention, on peut citer Li⁺, Na⁺, K⁺, Rb⁺, et Cs⁺

A titre d'exemples des cations des métaux alcalino-terreux, on peut citer Be²⁺, Mg²⁺, Ca²⁺, Sr²⁺, et Ba²⁺.

A titre d'exemples des cations des métaux de transition, on peut citer un cation de scandium, de titane, de vanadium, de chrome, de manganèse, de fer, de cobalt, de nickel, de cuivre, de zinc, de yttrium, de palladium, d'argent, de platine, et d'or.

Chaque molécule dudit complexe de molybdène de formule I peut comporter en outre plusieurs molécules d'eau de solvatation à l'état solide.

Un mode de réalisation de la présente invention concerne un complexe de molybdène de formule I(i) suivante :

A1ₘ-[Mo₂O₄]-Lₙ I(i)

dans laquelle :
- A1 est un cation choisi parmi les cations des métaux alcalins, les cations des métaux alcalino-terreux, les cations des métaux de transition, ou un cation organique PPh₄⁺,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou des larves par l'acarien *Varroa Destructor.*

Un autre mode de réalisation de l'invention concerne un complexe de molybdène de formule I(ii) suivante :

A1ₘ-[Mo₂O₂S₂]-Lₙ I(ii)

dans laquelle :
- A1 est un cation choisi parmi les cations des métaux alcalins, les cations des métaux alcalino-terreux, les cations des métaux de transition, ou un cation organique PPh₄⁺,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou des larves par l'acarien *Varroa destructor.*

Un mode de réalisation avantageux de l'invention concerne un complexe de molybdène de formule II suivante :

A2ₘ-[Mo₂O₂E₂]-Lₙ II

dans laquelle :
- A2 est un cation choisi parmi les cations des métaux alcalins, les cations des métaux alcalino-terreux, ou les cations des métaux de transition,
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), l'imino-diacétate (IDA²⁻),
- m est le nombre de cations et est choisi parmi 0, 1, 2, 3 et 4 ; n est le nombre de ligands et est choisi parmi 1 et 2,
pour une utilisation dans la prévention de l'infestation des abeilles et/ou de leurs larves par l'acarien *Varroa destructor.*

Les complexes de molybdène de formule II, qui ne comportent pas de cation organique (PPh₄⁺), mais uniquement des cations inorganiques choisis parmi les cations des métaux alcalins, les cations des métaux alcalino-terreux, ou les cations des métaux de transition, lorsque la présence des cations est nécessaire, présentent des effets bénéfiques plus significatifs pour les abeilles et/ou leurs larves, que ce soit pour la production du miel, la prolificité de la reine, ou pour la protection de leurs abeilles et/ou des larves contre l'attaque de *Varroa destructor.*

A titre d'exemples des cations inorganiques avantageux pour le complexe de molybdène de formule II, on peut citer Na⁺, K⁺, Li⁺, Ca²⁺, Mg²⁺, et Zn²⁺.

Dans un mode de réalisation particulier, l'invention est mise en oeuvre par les complexes de molybdène de formules I, I(i), I(ii), ou II dans lesquelles le ligand est choisi parmi la cystéine (L-cys²⁻), des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻), le nombre de ligands n étant choisi parmi 1 et 2, le nombre de cations m étant choisi parmi 1, 2, 3 et 4.

Dans un mode de réalisation plus particulier, l'invention est mise en oeuvre par les complexes de molybdène de formule II, dans laquelle :
- A2 est un cation choisi parmi Na⁺, K⁺, Li⁺, Ca²⁺, Mg²⁺, et Zn²⁺,
- E est choisi parmi O ou S,
- L est un ligand choisi parmi la cystéine (L-cys²⁻), des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 1, 2, 3 et 4 ;
- n est le nombre de ligands et est choisi parmi 1 et 2.

Dans un mode de réalisation particulier de l'invention, les complexes de molybdène de formule I pour une utilisation dans la prévention contre l'acarien *Varroa destructor* sont choisis parmi Na₂[Mo₂O₄(EDTA)].xH₂O (x = 5-6), Li₂[Mo₂O₄(EDTA)].xH₂O (x = 3-6), et (PPh₄)₂[Mo₂O₄(EDTA)].xH₂O (x = 2-5).

Afin de protéger efficacement les abeilles et/ou les larves contre l'acarien *Varroa destructor,* il convient d'utiliser au moins un complexe de molybdène de formule I, I(i), I(ii) ou II en une quantité annuelle de 1 à 60 mg/colonie d'abeilles, notamment de 1 à 40 mg/colonie d'abeilles, encore notamment de 1 à 20 mg/ colonie d'abeilles, encore notamment de 1 à 15 mg/ colonie d'abeilles, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

Ces quantités annuelles correspondent à un ordre de quelques micromoles du complexe de formule I, I(i), I(ii) ou II.

On entend par « une quantité annuelle » une quantité globale administrée à une colonie d'abeilles dans une durée de 12 mois successives.

L'administration peut être effectuée par une dose unique ou par plusieurs doses selon un protocole de nourrissement.

On entend par le terme « une colonie d'abeilles » un groupe d'abeilles qui peuple la ruche et formé par 3 types d'abeilles, à savoir une seule reine qui est une femelle sexuée, plusieurs milliers d'ouvrières qui sont des femelles non sexuées et quelques dizaines de faux bourdons qui sont des mâles.

Les termes « une colonie d'abeilles » et « un essaim d'abeilles » sont interchangeables.

Les complexes de molybdène de formules I, I(i), I(ii), ou II tels que décrits ci-dessus peuvent être utilisés dans la prévention contre l'infestation de *Varroa destructor* pour toutes races d'abeilles domestiques destinées à l'apiculture, notamment pour les races d'abeilles domestiques européennes.

Afin d'être administrés chez les abeilles, ledit complexe de molybdène de formule I, I(i), I(ii) ou II peut être incorporé dans un complément alimentaire des abeilles sous forme de poudre, d'un sirop, d'une pâte ou d'une solution aqueuse.

Un ou plusieurs complexes de molybdène de formule I, I(i), I(ii) ou II peuvent être mélangés avec un ou plusieurs aliments des abeilles, tels que de l'eau, du pollen, du nectar, du miel, des sucres, des vitamines, des minéraux, des lipides, et des protéines.

Dans un mode de réalisation particulier de l'invention, plusieurs différents complexes de molybdène de formule I, I(i), I(ii) ou II peuvent être utilisés en même temps.

Un autre aspect de la présente invention concerne une composition comprenant au moins un complexe de molybdène de formule I suivante :

A1ₘ-[Mo₂O₂E₂]-Lₙ I

dans laquelle :
- A1 est un cation choisi parmi les cations des métaux alcalins, les cations des métaux alcalino-terreux, les cations des métaux de transition, ou un cation organique PPh₄⁺
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou de leurs larves par l'acarien *Varroa destructor.*

La susdite composition peut comprendre en outre un ou plusieurs aliments pour les abeilles choisis parmi l'eau, le pollen, le nectar, le miel, les sucres, les vitamines, les minéraux, les lipides, et les protéines.

Ladite composition peut être sous forme d'une solution aqueuse, d'un concentré, d'une pâte, d'un sirop, ou sous forme de poudre soluble dans l'eau.

L'homme du métier peut adapter la forme de la composition selon la période, la fréquence et/ou l'objectif de nourrissement.

Dans un mode de réalisation plus particulier de la susdite composition pour la prévention de l'infestation des abeilles et/ou de leurs larves par l'acarien *Varroa destructor,* ledit complexe de molybdène correspond à la formule I(i) suivante :

A1ₘ-[Mo₂O₄]-Lₙ I(i)

dans laquelle :
- A1 est un cation choisi parmi les cations des métaux alcalins, les cations des métaux alcalino-terreux, les cations des métaux de transition, ou un cation organique PPh₄⁺,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2.

Dans un autre mode de réalisation plus particulier de la susdite composition pour la prévention de l'infestation des abeilles et/ou de leurs larves par l'acarien *Varroa destructor,* ledit complexe de molybdène correspond à la formule I(ii) suivante :

A1ₘ-[Mo₂O₂S₂]-Lₙ I(ii)

dans laquelle :
- A1 est un cation choisi parmi les cations des métaux alcalins, les cations des métaux alcalino-terreux, les cations des métaux de transition, ou un cation organique PPh₄⁺,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), l'imino-diacétate (IDA²⁻),
- m est le nombre de cations et est choisi parmi 0, 1, 2, 3 et 4 ;
- n est le nombre de ligands et est choisi parmi 1 et 2.
pour une utilisation dans la prévention de l'infestation des abeilles et/ou de leurs larves par l'acarien *Varroa destructor.*

Dans un mode de réalisation plus particulier de l'invention, ladite composition comprend un complexe de molybdène de formule I(i) choisi parmi Na₂[Mo₂O₄(EDTA)].xH₂O (x = 5-6), Li₂[Mo₂O₄(EDTA)].xH₂O (x = 3-6), et (PPh₄)₂[Mo₂O₄(EDTA)].xH₂O (x = 2-5).

Dans un autre mode de réalisation particulier de la susdite composition pour la prévention de l'infestation des abeilles et/ou de leurs larves par l'acarien *Varroa destructor,* ledit complexe de molybdène correspond à la formule II suivante :

A2ₘ-[Mo₂O₂E₂]-Lₙ, II

dans laquelle :
- A2 est un cation choisi parmi les cations des métaux alcalins, les cations des métaux alcalino-terreux, ou les cations des métaux de transition,
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻)
- m est le nombre de cations et est choisi parmi 0, 1, 2, 3 et 4 ;
- n est le nombre de ligands et est choisi parmi 1 et 2.

Dans un mode de réalisation plus particulier de l'invention, ladite composition comprend un complexe de molybdène de formule II comportant un cation choisi parmi Na⁺, K⁺, Li⁺, Ca²⁺, Mg²⁺, et Zn²⁺.

Dans un mode de réalisation encore plus particulier de l'invention, ladite composition comprend un complexe de molybdène de formule II, comportant un cation choisi parmi Na⁺, K⁺, Li⁺, Ca²⁺, Mg²⁺, et Zn²⁺, et un ligand L choisi parmi la cystéine (L-cys²⁻), des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻), le nombre de cations m étant choisi parmi 1, 2, 3 et 4 ; et le nombre de ligands n étant choisi parmi 1 et 2.

La présente invention a également pour objet un complexe ou une composition comprenant ledit complexe utilisés pour une méthode de prévention contre l'infestation des abeilles et de leurs larves par l'acarien *Varroa destructor.*

Ladite méthode comprend ou consiste en le nourrissement de la colonie avec un complexe de molybdène de formule I, I(i), I(ii), ou II, tel que défini auparavant ou une composition comprenant un complexe de molybdène de formule I, I(i), I(ii), ou II, tel que défini auparavant, la quantité globale annuelle dudit complexe utilisée par colonie étant de 1 à 60 mg/colonie d'abeilles, notamment de 1 à 40 mg/colonie d'abeilles, encore notamment de 1 à 20 mg/colonie, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

Ladite méthode de prévention peut être mise en oeuvre par une dose unique.

Dans un mode de réalisation particulier de ladite méthode, chaque colonie est nourrie par un complexe de molybdène de formule I, I(i), I(ii) ou II, tel que défini auparavant ou par une composition comprenant un complexe de molybdène de formule I, I(i), I(ii), ou II, tel que défini auparavant, à une dose unique de 1 à 60 mg/colonie d'abeilles, notamment de 1 à 40 mg/colonie d'abeilles, encore notamment de 1 à 20 mg, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

Ladite méthode de prévention peut aussi être mise en oeuvre par plusieurs doses selon un protocole afin d'atteindre la quantité globale annuelle d'un complexe de formule I, I(i), I(ii) ou II, de 1 à 60 mg/colonie d'abeilles, notamment de 1 à 40 mg/colonie d'abeilles, encore notamment 1 à 20 mg/colonie, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

La fréquence de nourrissement et la quantité du complexe pour chaque nourrissement peuvent être adaptées selon la quantité globale annuelle à administrer.

Par exemple, chaque colonie peut être nourrie par un complexe de molybdène de formule I, I(i), I(ii), ou II tel que défini auparavant ou par une composition comprenant un complexe de molybdène de formule I, I(i), I(ii), ou II, tel que défini auparavant, à une quantité d'un complexe de molybdène de 0,1 à 1 mg/colonie tous les deux jours pendant 20 jours, afin d'arriver à une quantité globale de 1 à 10 mg/colonie.

La période de la mise en oeuvre de ladite méthode de prévention peut être n'importe quelle période de l'année, mais de préférence la période s'écoulant entre l'automne et le printemps.

Par exemple, afin de préparer la colonie pour la période hivernale, il convient de nourrir en automne la colonie avec un sirop comprenant un complexe de molybdène de formule I, I(i), I(ii) ou II ou une composition telle que définie auparavant sous forme de sirop. Ce nourrissement peut être effectué dès que les températures descendent en dessous de 10°C environ.

Par ailleurs, pour stimuler les abeilles pour la nouvelle saison apicole, il est possible de nourrir les abeilles soit en janvier avec un pain de Candi comprenant un complexe de molybdène de formule I, I(i), I(ii) ou II ou une composition telle que définie auparavant sous forme d'un pain de candi, soit au début de printemps avec un sirop comprenant un complexe de molybdène de formule I, I(i), I(ii) ou II ou une composition telle que définie auparavant sous forme de sirop.

Le pain de candi pour le nourrissement en hiver peut être quantifié pour une consommation de plusieurs mois pendant la période d'hiver

Le nourrissement spéculatif qui se fait au début de printemps permet de simuler une miellée et d'avancer l'arrivée de la première génération d'abeilles de la saison. En général, le nourrissement spéculatif est effectué environ 30-40 jours avant l'arrivée supposée des premières fleurs dans la nature.

La présente invention a aussi pour objet un complément alimentaire pour les abeilles.

Ledit complément alimentaire comprend un complexe de molybdène de formule II suivante :

A2ₘ-[Mo₂O₂E₂]-Lₙ, II

dans laquelle :
- A2 est un cation choisi parmi les cations des métaux alcalins, les cations des métaux alcalino-terreux, ou les cations des métaux de transition,
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻)
- m est le nombre de cations et est choisi parmi 0, 1, 2, 3 et 4 ;
- n est le nombre de ligands et est choisi parmi 1 et 2.

On entend par « complément alimentaire pour les abeilles » une nourriture supplémentaire à la nourriture naturelle collectée par les abeilles, ledit complément alimentaire étant fourni aux abeilles par le biais de nourrissements adaptés. Les compléments alimentaires pour les abeilles comprennent les nutriments essentiels pour les abeilles afin de pallier les carences nutritionnelles des abeilles et/ou de fortifier les défenses immunitaires des abeilles.

Dans un mode de réalisation particulier, ledit complément alimentaire pour les abeilles de l'invention comprend un complexe de molybdène représenté par la formule II(i) A2ₘ-[Mo₂O₄]-Lₙ ou par la formule II(ii) A2ₘ-[Mo₂O₂S₂]-Lₙ, A2, L, m et n étant tels que définis pour la formule II.

Dans un autre mode de réalisation particulier, ledit complément alimentaire pour les abeilles de l'invention comprend un complexe de molybdène de formule II, II(i) ou II(ii), dans laquelle A2 est un cation choisi parmi Li⁺, Na⁺, K⁺, Ca²⁺, Mg²⁺ et Zn²⁺.

Dans un autre mode de réalisation particulier, ledit complément alimentaire pour les abeilles de l'invention comprend un complexe de molybdène de formule II, II(i) ou II(ii) choisi parmi Na₂[Mo₂O₄(EDTA)].xH₂O, Li₂[Mo₂O₄(EDTA)].xH₂O, K₂[Mo₂O₂S₂(LCys)₂].xH₂O, Na₂[Mo₂O₄(LCys)₂].xH₂O, K₂[Mo₂O₂S₂](HNTA)₂].xH₂O, K₂[Mo₂O₂S₂(EDTA)].xH₂O, K₂[MO₂O₄(HNTA)₂].xH₂O, Mg[Mo₂O₄(EDTA)].xH₂O, Ca[Mo₂O₄(EDTA)].xH₂O, et Zn[Mo₂O₄(EDTA)].xH₂O avec x compris généralement entre 2 et 6.

Ledit complément alimentaire comprend en outre un ou plusieurs aliments pour les abeilles choisis parmi l'eau, le pollen, le nectar, le miel, les sucres, les vitamines, les minéraux, les lipides, et les protéines, afin d'apporter les acides aminées, vitamines, acides gras et oligo-éléments indispensables à la santé de l'abeille.

Selon la façon d'effectuer le nourrissement, ledit complément alimentaire de l'invention peut être sous forme de poudre, d'un sirop, d'une pâte ou d'une solution aqueuse.

Par exemple, pour le nourrissement spéculatif qui se fait au printemps et qui sert à stimuler la ponte de la reine, le complément alimentaire de l'invention peut être sous forme d'un sirop de sucre. Pour le nourrissement de complément programmé au début de l'automne ou effectué en hiver, ledit complément alimentaire de l'invention peut être sous forme d'un produit pâteux de sucre.

Ledit complément alimentaire de l'invention peut être sous forme d'un concentré à diluer dans un sirop.

La quantité d'un complexe de molybdène de formule II, II(i) ou II(ii) dans un complément alimentaire de l'invention peut être adaptée selon le type de produit, le moyen de nourrissement, et la fréquence de nourrissement de façon à ce que la quantité globale annuelle dudit complexe utilisée par colonie soit de 1 à 60 mg/colonie, notamment de 1 à 40 mg/colonie, encore notamment 1 à 20 mg/colonie, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

La présente invention concerne également une méthode de nourrissement des abeilles, ladite méthode comprenant ou consistant en le nourrissement de la colonie des abeilles avec un complément alimentaire comprenant un complexe de molybdène de formule II, II(i) ou II(ii) tel que décrit auparavant, de façon à ce que la quantité globale annuelle dudit complexe utilisée par colonie soit de 1 à 60 mg/colonie, notamment de 1 à 40 mg/colonie, encore notamment de 1 à 20 mg/colonie, encore notamment de 1 à 10 mg/colonie d'abeilles, encore notamment de 1 à 8 mg/colonie d'abeilles, encore notamment de 1,5 à 8 mg/colonie d'abeilles, et plus particulièrement de 2 à 6 mg/colonie d'abeilles.

Ladite méthode peut être mise en oeuvre pendant toute période de l'année, mais de préférence en automne, afin de préparer la colonie pour l'hiver, ou au printemps pour stimuler les abeilles.

Par exemple, afin de préparer la colonie pour la période hivernale, il convient de nourrir en automne la colonie avec un sirop comprenant un complexe de molybdène de formule I, I(i), I(ii) ou II ou une composition telle que définie auparavant sous forme de sirop. Ce nourrissement peut être effectué dès que les températures descendent en dessous de 10°C environ.

Par ailleurs, pour stimuler les abeilles pour la nouvelle saison apicole, il est possible de nourrir les abeilles soit en janvier avec un pain de candi comprenant un complexe de molybdène de formule I, I(i), I(ii) ou II ou une composition telle que définie auparavant sous forme de pain de candi, soit au début de printemps avec un sirop comprenant un complexe de molybdène de formule I, I(i), I(ii) ou II ou une composition telle que définie auparavant sous forme de sirop.

Le pain de candi pour le nourrissement en hiver peut être quantifié pour une consommation de plusieurs mois pendant la période d'hiver.

Le nourrissement spéculatif qui se fait au début de printemps permet de simuler une miellée et d'avancer l'arrivée de la première génération d'abeilles de la saison. En général, le nourrissement spéculatif est effectué environ 30-40 jours avant l'arrivée supposée des premières fleurs dans la nature.

Sans vouloir se lier à une quelconque théorie de l'invention, ladite méthode pourrait renforcer le système immunitaire des abeilles et des larves, qui permet d'augmenter la production de miel, et notamment de baisser la mortalité hivernale lorsqu'elle est appliquée en automne.

Ainsi, la présente invention a également pour objet un complément alimentaire pour les abeilles comprenant un complexe de molybdène de formule II telle que définie précédemment, pour une utilisation dans la diminution de la mortalité des colonies d'abeilles, notamment de la mortalité hivernale.

Enfin, la présente invention a pour objet un complément alimentaire pour les abeilles utilisé pour augmenter la production de miel chez les abeilles, notamment la production de miel à la première récolte, comprenant un complexe de molybdène de formule II telle que définie précédemment.

La présente invention est illustrée plus en détails dans les figures et les exemples ci-après.

### Figures :

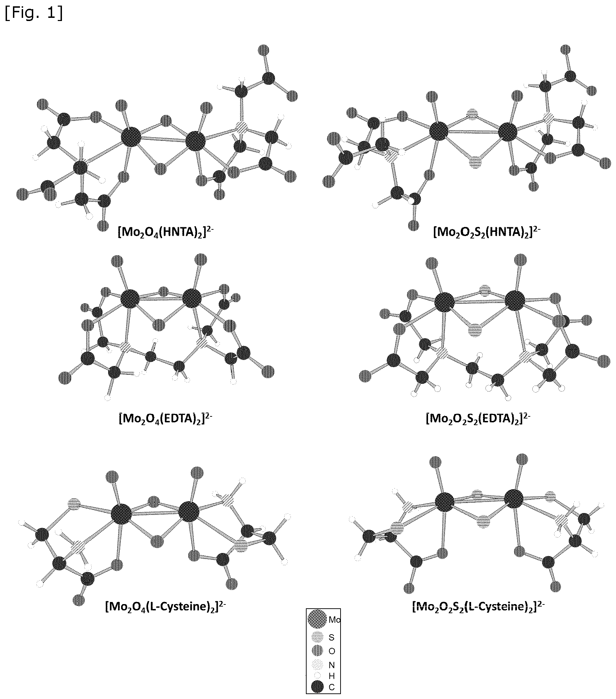

[Fig. 1] : Structures chimiques du cluster [Mo₂O₂S₂]²⁺ ou [Mo₂O₄]²⁺ complété par EDTA⁴⁻, HNTA²⁻, NTA³⁻, ou L-Cys²⁻. Dans la figure 1 : Mo (gris, hachure croisée), O (gris, hachure verticale), S (gris, hachure horizontale), N (gris, hachure oblique), C (noir), H (blanc).
[Fig. 2A] : Courbes de survie d'abeilles d'âges connus, en cagettes, nourries avec 20 mg/L de Na-Mo₂O₄-EDTA dans une solution sucrée alimentaire.
La ligne avec le symbole ■ correspond au groupe nourri avec une solution contenant Na-Mo₂O₄-EDTA.
La ligne avec le symbole □ correspond au groupe contrôle nourri avec une solution de saccharose.
Le trait pointillé indique le début du nourrissage avec la solution contenant Na-Mo₂O₄-EDTA pour le groupe traité. (20 mg/L : n= 50+/-3 pour chaque groupe).

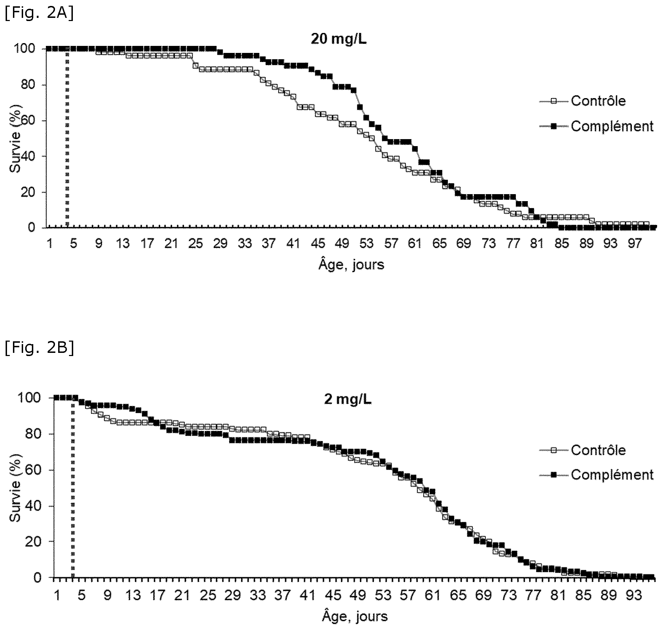

[Fig. 2B] : Courbes de survie d'abeilles d'âges connus, en cagettes, nourries avec 2 mg/L de Na-Mo₂O₄-EDTA dans une solution sucrée alimentaire.

La ligne avec le symbole ■ correspond au groupe nourri avec une solution contenant Na-Mo₂O₄-EDTA.

La ligne avec le symbole □ correspond au groupe contrôle nourris avec une solution de saccharose.

Le trait pointillé indique le début du nourrissage avec la solution contenant Na-Mo₂O₄-EDTA pour le groupe traité.

L'expérience a été répétée sur 3 groupes d'abeilles. Chaque groupe comporte 50 abeilles.

### Exemples

### 1. Synthèse des complexes de molybdène de formule I

Les méthodes de synthèse de deux complexes de formule I, Na₂[Mo₂O₄(EDTA)].5H₂O et Li₂[Mo₂O₄(EDTA)].6H₂O, sont décrites ci-après. La synthèse du composé (PPh₄)₂[Mo₂O₄(EDTA)].2,5H₂O, dénoté PPh₄-Mo₂O₄-EDTA, a été divulguée dans le brevet MD4438.

### Synthèse de Na₂[Mo₂O₄(EDTA)]_{.5}H₂O, dénoté Na-Mo₂O₄-EDTA; Mm = 680,15 g/mol

Du Molybdate de sodium dihydraté Na₂MoO₄*2H₂O (2,92 g, 12.07 mmol) et du Na₂H₂EDTA*2H₂O (2,25 g, 6,04 mmol) sont dissous dans 30 mL d'eau distillée. Une solution aqueuse de sulfate d'hydrazinium N₂H₄*H₂SO₄ (785,96 mg, 6.04 mmol / 10 mL d'eau) est ajoutée et une solution de HCl 1M est utilisée pour ajuster le pH à 3 afin de permettre la réduction de [Mo^{(VI)}O₄]²⁻ en [Mo^{(V)}₂O₄]²⁺. La solution rouge foncé obtenue est chauffée à 80 °C pendant une heure et demie avec agitation continue. Après refroidissement à température ambiante, la solution est transférée dans un cristallisoir et est laissée évaporer. Après quelques jours, des cristaux rouges du produit pur **Na₂[Mo₂O₄(EDTA)].5H₂O** sont collectés par filtration, lavés rapidement à l'eau froide et laissés sécher à l'air. Le rendement est alors de 65%. Le produit est caractérisé par FT-IR, analyse élémentaire, ATG, ESI-MS, EDX et RMN. FT-IR (v, cm⁻¹): 3518 (m), 3300 (m), 1645 (s), 1445 (w), 1384 (m), 1350 (s, sh.), 1243 (w), 972 (w), 944 (m), 911 (w), 863 (w), 761 (w); Analyse élémentaire pour **Na₂[Mo₂O₄(C₁₀H₁₂N₂O₈)].5H₂O** Calculé (trouvé): C 17.66 (17.42) H 3.26 (2.92) N 4.12 (4.18); EDX : ratio atomique attendu (trouvé) Mo/Na 1.00 (1.05); ESI-MS m/z 272.05 (m/z calculé pour [Mo₂O₄(EDTA)₂]²⁻ 271.93); l'analyse ATG montre 13.3 % perte de poids dans la plage de température de 25-200 ° C correspondant à l'eau d'hydratation (calculé 13.2 %); ¹H-NMR (δ ppm, 400 MHz, D₂O) 2.61 (m, large, 4H), 3.40 (m, large, 8H).

### Synthèse de Li₂[Mo₂O₄(EDTA)]_{.6}H₂O, dénoté Li-Mo₂O₄-EDTA; Mm = 666.07 g/mol

Le composé **Li₂[Mo₂O₄(EDTA)].6H₂O** est obtenu par échange des cations Na⁺ du composé **Na₂[Mo₂O₄(EDTA)].5H₂O** par Li⁺ sur une résine échangeuse de cations DOWEX chargée en cations Li⁺. Pour ce faire, 1 g de **Na₂[Mo₂O₄(EDTA)].5H₂O** est dissous dans un volume minimum d'eau (10 mL) et cette solution concentrée est passée sur une colonne chargée en résine. Une solution aqueuse du sel de lithium **Li₂[Mo₂O₄(EDTA)]** est obtenue. Cette dernière est vaporisée à sec sous pression réduite à l'évaporateur rotatif pour obtenir le produit solide désiré. Rendement 90%. Le produit est caractérisé par FT-IR, analyse élémentaire, ATG, ESI-MS, EDX et RMN. (η = 90 %). FT-IR (v, cm⁻¹): 3412 (s, br.), 2983 (w), 1651 (s), 1625 (s), 1446 (w), 1392 (m), 1357 (m), 1244 (w), 964 (m), 948 (w), 933 (w), 909 (w), 861 (w), 759 (w), 475 (w); Analyse élémentaire pour **Li₂[Mo₂O₄(C₁₀H₁₂N₂O₈)].6H₂O** Calculé (trouvé): C 18.03 (17.79) H 3.63 (3.38) N 4.21 (4.10); EDX : ratio atomique attendu (trouvé) Mo 100% (100%). Pas de trace de Na prouvant que l'échange de cation a bien eu lieu ; ESI-MS m/z 272.05 **[Mo₂O₄(EDTA)₂]²⁻** (271.92); L'analyse ATG montre 14.83 % perte de poids dans la plage de température de 25-200 ° C correspondant à l'eau d'hydratation (calculé 13.9 %); ¹H-NMR (δ ppm, 300 MHz, D₂O) 2.54 (m, large, 4H), 3.33 (m, large, 8H).

### 2. Toxicité des complexes de formule I

La toxicité des complexes de formule I a été évaluée par 3 méthodes différentes : tests *in vitro* sur cellules, sur protozoaires et directement sur abeilles.

### 2.1 Étude de cytotoxicité in vitro

Pour la culture cellulaire, des cellules épithéliales cervicales humaines de la lignée HeLa, des cellules d'adénocarcinome pancréatique épithélial humain de la lignée BxPC-3 et des cellules épithéliales saines Madin Darby Canine Kidney de la lignée MDCK ont été utilisées dans cette étude. Le test Resazurin de prolifération cellulaire a été appliqué selon la littérature (S. Anoopkumar-Dukie, J. B. Carey, T. Conere, E. O'sullivan, F. N. van Pelt, A. Allshire, Br J Radiol 2005, 78, 945, DOI: 10.1259/bjr/54004230). Comme indicateur de l'efficacité des composés expérimentaux sur la prolifération des cellules, la concentration inhibitrice médiane (CI₅₀) a été utilisée. Plus la valeur CI₅₀ est faible, plus l'activité (dans ce cas la cytotoxicité) est élevée. Pour des valeurs CI₅₀ égales et supérieures à 100 µM, il est considéré qu'il n'y a pas d'activité.

Cinq complexes de formule I sont testés dans cette étude en comparaison avec deux composés anti-cancéreux de référence (complexe « Cis-platine » et Doxorubicine).

Les résultats des tests sont donnés dans le tableau 1 ci-après.

**[Tableau 1]**

| Composé | CI₅₀, µM | | |
|---|---|---|---|
| | MDCK | BxPc-3 | HeLa |
| PPh₄-Mo₂O₄-EDTA | 60,72 ± 12,1 | 25,09 ± 7,02 | ≥ 100 |
| Na-Mo₂O₄-EDTA | Inactif | Inactif | Inactif |
| Li-Mo₂O₄-EDTA | Inactif | Inactif | Inactif |
| PPh₄-Mo₂O₂S₂-EDTA | ≥ 100 | 59,81 ± 26,45 | ≥ 100 |
| K- Mo₂O₂S₂-Cys | Inactif | Inactif | Inactif |
| Cis-platine (ref. 1) | 30,9 ± 1,1 | 11,2 ± 1,2 | 3,99 ± 0,33 |
| Doxorubicine (ref. 2) | 1,48 ± 3,1 | 3,99 ± 7,9 | 1,31 ± 4,9 |

| | | | |
|---|---|---|---|
| Cytotoxicité des composés sur les cellules saines (MDCK), le cancer du pancréas (BxPc-3) et les cellules du cancer du col utérin (HeLa). | | | |

Le tableau 1 démontre que les composés étudiés sont non cytotoxiques pour la plupart. Les composés avec les contre cations PPh₄⁺ montrent une faible activité liée au cation organique lui-même. Néanmoins, les CI₅₀ donnent des concentrations au moins 100 fois supérieures à celles utilisées dans les ruches (de l'ordre de 0,2-0,6 µM). Les sels Na-Mo₂O₄-EDTA et Li-Mo₂O₄-EDTA n'ont aucune cytotoxicité.

### 2.2 Étude de toxicité sur protozoaires.

Cette étude a été menée aussi par les chercheurs du Département de Chimie de l'Université d'Etat de Moldavie. Les composés qui n'ont montré aucune toxicité *in vitro* sur les cellules ont été utilisés dans d'autres tests sur des micro-organismes monocellulaires simples, à savoir les protozoaires. La méthode spectrophotométrique a été utilisée pour évaluer la toxicité des substances biologiquement actives par rapport à une culture de paramécie utilisant le colorant NR, selon le protocole décrit dans la littérature (1. Zhang, S.Z., Lipsky, M.M., Trump, B.F., Hsu, I.C. Neutral red (NR) assay for cell viability and xenobiotic-induced cytotoxicity in primary cultures of human and rat hepatocytes. Cell Biol Toxicol. 1990;6(2):219-34; 2. Repetto, G., del Peso, A., Zurita, J.L.. Neutral red uptake assay for the estimation of cell viability/cytotoxicity. Nat Protoc 3: 1125-1131.). À la fin, la valeur CL₅₀ (concentration létale médiane) est déterminée. Les résultats des tests sont donnés dans le tableau 2.

**[Tableau 2]**

| Composé | CL₅₀, µM | |
|---|---|---|
| | 24 h | 48 h |
| PPh₄-Mo₂O₄-EDTA | ≥ 100 | ≥ 100 |
| Na-Mo₂O₄-EDTA | ≥ 100 | ≥ 100 |
| Li-Mo₂O₄-EDTA | ≥ 100 | ≥ 100 |
| PPh₄-Mo₂O₂S₂-EDTA | 40,25 ± 5,5 | ≥ 100 |
| K- Mo₂O₂S₂-Cys | ≥ 100 | ≥ 100 |

### Toxicité des composés sur les micro-organismes protozoaires Paramecium

Une CL₅₀ inférieure à 100 µM reflète une forte toxicité. En revanche, une valeur ≥100 indique qu'il n'y a aucun effet toxique sur le développement des micro-organismes *Paramecium.* Des 5 composés testés, seul un présente une légère activité qui disparait rapidement. Les 5 composés testés sont jugés non toxiques.

### 2.3 Étude de toxicité sur des abeilles.

Des tests complémentaires sur l'impact de la durée de vie des abeilles ont été effectués.

Cinquante abeilles fraîchement nées (âge = 1 jour) sont placées dans une cagette spéciale avec une bande de cire, de l'eau et de la nourriture, et sont maintenues dans l'étuve de laboratoire, à température constante de 33° C et 50% d'humidité. Un groupe témoin d'abeilles est nourri par une solution contenant uniquement du saccharose ; un groupe d'abeille est nourri par une solution contenant du saccharose et 2 mg/L de Na-Mo₂O₄-EDTA ; un autre groupe d'abeilles est nourri par une solution contenant du saccharose et 20 mg/L de Na-Mo₂O₄-EDTA. La consommation d'eau et de nourriture est contrôlée chaque jour. Les abeilles mortes sont comptées et retirées. Pour des raisons statistiques, les expériences sont répétées jusqu'à 3 fois si nécessaire.

Il n'y a pas de différence significative entre le groupe témoin et le groupe traité qui est nourri respectivement par une solution contenant 2 mg/L de Na-Mo₂O₄-EDTA (p>0,05, X² = 3,36, ddl=1) (Figure 2B) ou une solution contenant 20 mg/L de Na-Mo₂O₄-EDTA (p>0,05, X² = 0,04, ddl=1) (Figure 2A).

Cette étude confirme que Na-Mo₂O₄-EDTA n'induit pas de mortalité d'abeilles à 2 mg/L ni à 20 mg/L.

Ce test montre sans ambiguïté la non-toxicité du complexe [Mo₂O₄(EDTA)]²⁻ sur les abeilles.

### 3. Stabilité chimique des complexes de formule I

A l'état solide, les composés obtenus sont parfaitement stables à l'air et peuvent être conservés sans condition particulière pendant des années sans aucune altération.

Les stabilités des complexes en solution sont étudiées par différentes techniques (spectroscopie ¹H-RMN ou UV-vis), l'objectif étant d'étudier leur comportement dans différents milieux en termes de stabilité.

Les expériences de stabilité dans l'eau dans la gamme de concentrations 5×10⁻³ - 2×10⁻⁷ M révèlent le fait que les complexes d'EDTA ([Mo₂O₂S₂-EDTA]²- et [Mo₂O₄-EDTA]²⁻) ne montrent aucun signe de décomposition dans l'eau, ce qui suggère une très forte stabilité chimique. Dans le cas de K-Mo₂O₂S₂-LCys, on peut descendre également jusqu'à des concentrations micromolaires sans décomposition, ce qui suggère également une bonne stabilité chimique. Cela signifie que ces composés ne subissent pas de modifications structurelles dans des conditions de dilution importantes. Les solutions aqueuses de ces complexes s'avèrent particulièrement stables dans le temps.

En milieu physiologique ou dans un sirop de sucre à 50% en masse, les complexes [Mo₂O₂S₂-EDTA]²⁻ et [Mo₂O₄-EDTA]²- (sels de Na et Li) et K-Mo₂O₂S₂-L-Cys montrent également une bonne stabilité à très faible concentration.

En conclusion, les complexes de formule I s'avèrent particulièrement stables dans différents milieux et à forte dilution.

### 4. Tests des complexes de formule I dans les ruches

### 4.1. 1^{er} test dans les ruches

### Protocoles employés, paramètres suivis et méthodes employées

Les tests biologiques sur terrain ont été effectués sur des familles d'abeilles domestiques européennes de race *Apis Mellifera Carpatica* dans un rucher expérimental situé dans le district forestier de Ghidighici (République de Moldavie). Le nourrissement avec le complexe de l'invention a eu lieu au printemps. Les tests biologiques sont suivis jusqu'au mois de septembre.

Pour administrer les composés aux abeilles, une très petite quantité de substance bioactive est ajoutée à deux types de produits de nourrissement sucrés pour les abeilles : des candi sous forme d'une pâte (70% saccharose, 30% miel) ou du sirop (50% saccharose, 50% eau). L'alimentation des abeilles se fait en ajoutant le candi/sirop dans le nourrisseur situé dans la partie supérieure intérieure de la ruche. Le volume de sirop est ajusté en fonction du nombre de cadres d'abeilles au moment initial, plus précisément 200 g de candi par cadre et 100 mL de sirop par cadre, soit 2kg de Candy et 1 L de sirop par ruche de 10 cadres.

Pour la première étape de l'alimentation, le candi sous forme d'une pâte n'est donné qu'une seule fois. Ensuite, pour l'alimentation principale, des volumes déterminés de sirop sont utilisés à plusieurs reprises pour nourrir les abeilles tous les deux jours pendant une période de deux semaines, ce qui correspond à un nourrissement d'une quantité annuelle d'environ 2 mg à 6 mg de chaque composé pour chaque ruche. Du début du test jusqu'à la fin (après la deuxième récolte), toutes les familles d'abeilles sont surveillées pour plusieurs paramètres morpho-producteurs clés qui reflètent le niveau de développement de la colonie et les aspects vitaux des abeilles. Les paramètres étudiés sont : la prolificité de la reine (fécondité), la puissance des colonies, la production de miel et la production de cire. Le suivi de ces paramètres est en accord avec les normes zootechniques concernant l'évaluation des familles d'abeilles, l'élevage et la certification du matériel parental apicole approuvé par la décision gouvernementale Nr 306 du 28/04/2011 de la République de Moldavie.

### Évaluation de la production de miel :

La production de miel (en kg) est déterminée pour chaque famille d'abeilles en additionnant la quantité de miel-marchandise (miel des hausses), extraite pendant la saison de récolte, avec la quantité de miel accumulée dans le nid (corps de ruche) et laissée comme nourriture pour les abeilles pour la période d'hiver (miel du couvain). La quantité de miel-marchandise sera déterminée pour chaque famille, à chaque extraction, en pesant des cadres/rayons de miel avant et après extraction (avec une précision de 0,1 kg), la différence de poids constituant la quantité de miel-marchandise extraite. La quantité de miel laissée dans le nid pour l'alimentation des abeilles est déterminée par la visite d'automne (septembre), en pesant les cadres de miel et en diminuant (à partir de leur poids total) le poids total des cadres en nid d'abeilles standard : pour le cadre de type Dadant (435x300 mm) - 0,6 kg, pour cadre Langstroth (435x230 mm) - 0,5 kg.

### Évaluation de la puissance de la colonie :

La puissance de la colonie d'abeilles représente la quantité d'abeilles existant dans le nid au moment de l'appréciation. L'évaluation est effectuée trois fois par an : à la visite de printemps (mars-avril), à la fin du printemps (20-31 mai) et à la visite d'automne (septembre). Suite à ces trois évaluations, la puissance moyenne de la famille d'abeilles est déterminée. La quantité d'abeilles (kg) est déterminée en multipliant le nombre d'intervalles entre les cadres, uniformément occupés par les abeilles, avec le coefficient 0,25 pour le cadre standard Dadant (435x300 mm) et 0,2 pour le cadre Langstroth standard (435x230 mm).

### Évaluation de la prolificité de la reine :

La prolificité de la reine (oeufs/24 heures) est déterminée lors de la visite à la fin du printemps (20-31 mai) en divisant le nombre de cellules avec du couvain par 12 (durée du développement du couvain, jours), le nombre d'oeufs pondus dans les 24 heures. Le nombre de cellules dans le nid est déterminé en mesurant avec le cadre Netz le nombre de carrés (5 × 5 cm) occupés par le couvain qui est multiplié par 100, ce qui donne le total nombre de cellules avec le couvain couvert.

### Évaluation du degré d'infestation avec Varroa

L'évaluation consiste à compter le nombre de varroas sur un échantillon d'abeilles adultes. La valeur issue de ce comptage constitue un indice pour le suivi du niveau de parasitisme de la colonie.

La méthode utilise du sucre glace et un pot « shaker » en verre transparent d'une capacité de 1kg. Le couvercle est un grillage en acier galvanisé de type grillage de fond de ruche, de maille 3 mm. Le grillage laisse passer les varroas mais retient les abeilles.

La méthode est mise en oeuvre selon les étapes suivantes : On prélève 40 à 50 g d'abeilles dans le bocal (environ 400 abeilles), on ajoute 100 g de sucre glace et on roule le pot shaker sur lui-même pendant 1 minutes de manière à recouvrir de sucre glace toutes les abeilles. Le sucre glace permet alors de désolidariser les varroas du corps de l'abeille hôte et ils tombent. Laisser reposer 1 minute : les abeilles ont un comportement d'épouillage, qui renforce la chute des varroas. On tamise le sucre glace sur un tamis qui permet de retenir les varroas puis on les compte. Le résultat est ramené en nombre d'acariens pour 10 g d'abeilles (environ 100 abeilles) et peut être exprimé en %. Ce taux est indicatif de la pression en Varroa dans la colonie. Un taux entre 1 et 2 indique une faible pression en Varroa. Un taux modéré est compris entre 2 et 5 et dans ce cas un traitement des colonies est à programmer. Un taux, enfin supérieur à 5 nécessite un traitement d'urgence de la colonie. Dans le cas de nos essais nous sommes autour de 2% pour les abeilles ouvrières.

### Résultats

Les molécules testées, leurs concentrations en solution et leurs doses globales annuelles sont listées ci-après :
1. (PPh₄)₂[Mo₂O₄(EDTA)].2,5H₂O, abrévié **PPh₄-Mo₂O₄-EDTA.**
Le nourrissement est effectué avec 2000 g de candi contenant 0,8 mg de complexe puis 6 à 7 L de sirop contenant 0,2 mg/L de complexe. La concentration de complexe dans le sirop : 1,5×10⁻⁷ mol/L. Dose globale et annuelle de produit : 2 mg de complexe par colonie
2. Na₂[Mo₂O₄(EDTA)].5H₂O, abrévié **Na-Mo₂O₄-EDTA.**
Le nourrissement est effectué avec 2000 g de candi contenant 0,8 mg de complexe puis 6 à 7 L de sirop contenant 0,2 mg/L de complexe. La concentration de complexe dans le sirop : 3,0×10⁻⁷ mol/L. Dose globale et annuelle de produit : 2 mg de complexe par colonie
3. Li₂[Mo₂O₄(EDTA)].6H₂O, abrévié **Li-Mo₂O₄-EDTA.**
Le nourrissement est effectué avec 2000 g de candi contenant 0,8 mg de complexe puis 6 à 7 L de sirop contenant 0,2 mg/L de complexe. La concentration de complexe dans le sirop : 3,2×10⁻⁷ mol/L ; la concentration en Lithium C(Li) = 6,4×10⁻⁷ mol/L soit 4,5×10⁻⁶ g/L. Dose globale et annuelle de produit : 2 mg de complexe par colonie
4. LiCH₃COO.H₂O comme un témoin positif, abrévié Li-Ac
Le nourrissement est effectué avec 2000 g de candi contenant 0,8 mg de produit de témoin positif puis 6 à 7 L de sirop contenant 0,2 mg/L de produit de témoin positif. La concentration de produit dans le sirop : 2,36×10⁻⁶ mol/L soit 1,67 ×10⁻⁵ g/L. Dose globale et annuelle de produit : 2 mg de produit par colonie
Les résultats sont illustrés dans le tableau 3 ci-après.

**[Tableau 3]**

| Caractères morpho-productifs | Lot I, témoin | Lot II, **PPh₄**-**Mo₂O₄**-**EDTA** | Lot III, **Na-Mo₂O₄**-**EDTA** | Lot IV, **Li-Mo₂O₄**-**EDTA** | Lot V, Li-Ac |
|---|---|---|---|---|---|
| | (N* = 10) | Dose globale 2 mg (N* = 10) | Dose globale 2 mg (N* = 10) | Dose globale 2 mg (N* = 10) | Dose globale 2 mg (N* = 10) |
| | % | % par rapport au lot I | % par rapport au lot I | % par rapport au lot I | % par rapport au lot I |
| Prolificité de la reine | 100 | 107,1 | 113,3 | 111,7 | 108,8 |
| Puissance de la famille d'abeilles | 100 | 102,1 | 101,2 | 103,3 | 98,3 |
| Quantité de miel à la première récolte | 100 | 113,3 (+13,3 %) | 149,9 **(+49,9 %)** | 143,1 **(+43,1%)** | 122,9 (+22,9 %) |
| Degré d'infestation avec *Varroa* | 100^{a} | 78,7 (-21,3%) | 86,5 (-13,5%) | 57,3 (-42,7%) | 58,4 (-41,6%) |

| | | | | | |
|---|---|---|---|---|---|
| N* - nombre de ruches, a) taux d'infestation initial moyen de 1,78 acarien /10 g d'abeilles (100 abeilles environ) soit 1,78%. Cette valeur est amenée à 100 en guise de référence pour nos essais. | | | | | |

Nous pouvons constater que les trois complexes de molybdène de formule I montrent un effet protecteur significatif contre l'infestation des abeilles par *Varroa destructor* par rapport au lot I non traité.

A une concentration de lithium presque 4 fois inférieure, le complexe Li-Mo₂O₄-EDTA montre une capacité protective comparable à celle de l'acétate de lithium employé comme témoin positif. Cette quantité de lithium est largement inférieure à la quantité minimale efficace de 2 mM utilisée dans la publication de B. Ziegelmann *et al.* (*Scientific Reports* 2018, 8, DOI: https://doi.org/10.1038/s41598-017-19137-5).

Par ailleurs, deux molécules de formule II testées (Na-Mo₂O₄-EDTA et Li-Mo₂O₄-EDTA) montrent des effets supérieurs par rapport à PPh₄-Mo₂O₄-EDTA, tant pour la prolificité de la reine que la quantité de miel récolté.

Les miels produits au printemps et en été pour les lots 1 à 4 ont été analysés dans un laboratoire accrédité COFRAC. Les résultats de ces analyses montrent que la composition du miel n'est pas altérée par le traitement avec les complexes de l'invention. Les miels produits sont conformes aux normes européennes et aucune trace de Mo n'est retrouvée dans le miel, donc une absence totale des complexes dans le miel produit.

### 4.2. 2^{ème} test dans les ruches

Le 2^{ème} test a été effectué dans le même rucher dans l'année suivante. Le nourrissement avec le complexe de l'invention a eu lieu au printemps. Les tests biologiques sont suivis jusqu'au mois de septembre.

Les molécules testées, leurs concentrations en solution et leurs doses globales annuelles sont listées ci-après :
1. (PPh₄)₂[Mo₂O₄(EDTA)].2,5H₂O, abrévié PPh₄-Mo₂O₄-EDTA,
Le nourrissement est effectué avec 2000 g de candy contenant 0,8 mg de complexe puis 6 à 7 L de sirop contenant 0,2 mg/L en complexe. La concentration de complexe dans le sirop: 1,5×10⁻⁷ mol/L. Dose globale de produit : 2 mg/colonie
2. Li₂[Mo₂O₄(EDTA)].6H₂O, abrévié Li-Mo₂O₄-EDTA.
Le nourrissement est effectué avec 2000 g de candy contenant 0,8 mg de complexe puis 6 à 7 L de sirop contenant 0,2 mg/L en complexe. La concentration de complexe dans le sirop: 3,2×10⁻⁷ mol/L ; la concentration en Lithium C(Li) = 6.4×10⁻⁷ mol/L soit 4,5×10⁻⁶ g/L; Dose globale de complexe : 2 mg/colonie
3. Li₂[Mo₂O₄(EDTA)].6H₂O, abrévié Li-Mo₂O₄-EDTA.
Le nourrissement est effectué avec 2000 g de candy contenant 2,4 mg de complexe puis 6 L de sirop contenant 0,6 mg/L en complexe. La concentration de complexe dans le sirop: 9,6×10⁻⁷ mol/L ; la concentration en Lithium C(Li) = 1,92×10⁻⁶ mol/L soit 1.32×10⁻⁵ g/L. Dose globale de complexe : 6 mg/ colonie
4. LiCH₃COO.H₂O pour comparaison, abrévié Li-Ac
Le nourrissement est effectué avec 2000 g de candy contenant 0,8 mg de produit puis 6 à 7 L de sirop contenant 0,2 mg/L en produit. La concentration de produit dans le sirop: 2,36×10⁻⁶ mol/L soit 1,67×10⁻⁵ g/L. Dose globale de produit : 2 mg/colonie
Les résultats sont illustrés dans le tableau 4 ci-après.

**[Tableau 4]**

| Caractères morpho-productifs | Lot I, témoin | Lot II, **PPh₄**-**MO₂O₄**-**EDTA** | Lot III, **Li-Mo₂O₄**-**EDTA** | Lot IV, **Li-Mo₂O₄**-**EDTA** | Lot V, Li-Ac |
|---|---|---|---|---|---|
| | (N* = 10) | Dose globale 2 mg/Ruche (N* = 10) | Dose globale 2 mg/Ruche (N* = 10) | Dose globale 6 mg/Ruche (N* = 10) | Dose globale 2 mg/Ruche (N* = 10) |
| | % | % par rapport au lot I | % par rapport au lot I | % par rapport au lot I | % par rapport au lot I |
| Puissance de la famille d'abeilles | 100 | 120 | 120 | Non mesuré | 97,5 |
| Degré d'infestation avec Varroa | 100^{a} | 60,8 (-39,2%) | 52,6 (-47,4%) | 38,4 (-62,6 %) | 69,0 (-31%) |
| **Degré d'infestation des larves avec Varroa** | **100^{b}** | **56,0 (-44,0%)** | **42,6 (-57,4%)** | **18,4 (-81,6%)** | **64,9 (-35,1%)** |

| | | | | | |
|---|---|---|---|---|---|
| N* - nombre de ruches, a) taux d'infestation initial moyen de 2,32 acarien /10 g d'abeilles (100 abeilles environ) soit 2,32% ; b) cette valeur référence de 100 correspond à un taux d'infestation initial moyen des larves de 28,2 % mesuré par comptage direct des larves infectées après ouverture d'une centaine d'alvéoles par ruche. | | | | | |

Les résultats du 2^{ème} test dans les ruches confirment les effets des complexes de formule I de l'invention dans la protection des abeilles contre l'infestation avec *Varroa.* D'ailleurs, selon les résultats illustrés dans le tableau 4, cet effet protecteur est accru avec l'augmentation de la dose.

Les résultats du 2^{ème} test montrent surtout que les complexe de formule I de l'invention permettent également de protéger les larves contre *Varroa.* A ce jour, il n'existe aucun traitement connu pour protéger les larves contre *Varroa.*

### 4.3. 3^{ème} test dans les ruches

Une campagne de traitement a été réalisée en automne-hiver dans des ruchers qui se situent en Californie, à côté de San Francisco. Cette région est particulièrement sensible à la mortalité hivernale des colonies d'abeilles et celle-ci peut atteindre jusqu'à 80% certaines années.

151 ruches, réparties sur 6 ruchers différents en Californie au sud de San Francisco ont été choisies. Les abeilles sont de différents types : *Wildflower* (Italienne VSH), *Beeweaver,* Abeille californienne carniolienne (*Pope Canyon*), Abeille californienne *Tom* et Abeille californienne Italienne (*Sam*)*.*

Les ruches sont des ruches de type Langstroth 2 corps (15 cadres contre 10 pour les ruches Dadant en Europe). Sur chaque rucher, on divise aléatoirement en deux populations de ruches celles qui servent de témoins et celles qui reçoivent le produit **Li-Mo₂O₄-EDTA** (76 témoins, 75 ruches test).

Le produit **Li-Mo₂O₄-EDTA** est introduit entre le 25 et le 28 octobre 2019 à la seringue sous forme d'une solution aqueuse concentrée (8 g/L) dans un sirop de sucre dans le nourrisseur cadres. 0,5 mL de cette solution sont introduits et dispersés dans 3,5 litres de sirop de sucre à 65% en sucre introduit dans la ruche. Chaque ruche test reçoit ainsi une dose unique globale de 4 mg de Li-Mo₂O₄-EDTA. Les ruches témoins ne reçoivent que le sirop de sucre.

Le décompte des colonies survivantes est effectué le 7 janvier 2020. Un total de 73 colonies sur les 151 initiales sont mortes, soit une mortalité globale de 48,3 %.

La mortalité des ruches témoins s'élève à 47 colonies perdues sur 76, soit 61,8 % de mortalité hivernale, qui est un résultat classique dans cette région.

La mortalité des ruches test traitées par 4 mg de Li-Mo₂O₄-EDTA s'élève à 26 colonies perdues sur les 75 initiales, soit 34,7% de mortalité hivernale, qui est une baisse très significative par rapport aux ruches témoins.

En conclusion, le complexe de molybdène de la présente invention permet de baisser significativement la mortalité hivernale des colonies d'abeilles.

### 4.4. 4^{ème} test dans les ruches

Une seconde campagne de traitement a été réalisée en automne-hiver 2020 dans des ruchers qui se situent en Californie, à côté de San Francisco. Cette région est particulièrement sensible à la mortalité hivernale des colonies d'abeilles et celle-ci peut atteindre jusqu'à 80% certaines années.

220 ruches réparties sur 13 ruchers différents en Californie au sud de San Francisco ont été choisies. Chaque rucher comprend entre 8 et 24 ruches. Les abeilles sont de différents types : 165 ruches d'abeilles *Wildflower*(Italienne VSH) et 55 ruches d'abeilles *Beeweaver* (Carnolienne VSH).

Les ruches sont des ruches de type Langstroth 2 corps (15 cadres contre 10 pour les ruches Dadant en Europe). Sur chaque rucher, on divise aléatoirement en 4 lots de ruches pour arriver à un total de :
- Lot1 : 55 ruches témoins
- Lot 2 : 55 ruches qui reçoivent **Li-Mo₂O₄-EDTA** le 20 septembre 2020
- Lot 3 : 55 ruches qui reçoivent **Li-Mo₂O₄-EDTA** le 12 octobre 2020
- Lot 4 : 55 ruches qui reçoivent **Li-Mo₂O₄-EDTA** le 20 septembre 2020 et le 12 octobre 2020.

1 rucher ne contient que des abeilles de type *Wildflower.* 2 ruchers ne contiennent que des abeilles de type *Beeweaver.* Les 10 autres ruchers comprennent des abeilles *Wildflower*et *Beeweaver* qui sont réparties équitablement entre les 4 lots.

Le produit **Li-Mo₂O₄-EDTA** a été introduit le 20 septembre 2020 ou/et le 12 octobre 2020 à la seringue sous forme d'une solution aqueuse concentrée (0,8 g/L) dans un sirop de sucre dans le nourrisseur cadres. 10 mL de cette solution sont introduits et dispersés dans 3,5 litres de sirop de sucre à 65% en sucre introduit dans la ruche. Chaque ruche test reçoit ainsi une dose unique globale de 8 mg de Li-Mo₂O₄-EDTA par nourrissement. Les ruches témoins ne reçoivent que le sirop de sucre.

Le décompte des colonies mortes est effectué le 31 décembre 2020. Un total de 28 colonies sont mortes au 31/12/2020, soit 12,73 % du total. Le taux de mortalité est identique pour les abeilles *Wildflower*(21/165) et *Beeweaver* (7/55). La mortalité par lot se répartie comme suit :
- Lot1 : 15 ruches / 55 ruches, soit 27,3 % de pertes
- Lot 2 : 0 ruche / 55 ruches, soit 0% de pertes
- Lot 3 : 10 ruches / 55 ruches, soit 18,2 % de pertes
- Lot 4 : 3 ruches / 55 ruches, soit 5,5 % de pertes.

En conclusion, le complexe de molybdène de la présente invention permet de baisser de manière très significative la mortalité hivernale des colonies d'abeilles en Californie pour une dose globale de 8 mg par colonie. Un nourrissement suffisamment tôt en septembre permet d'obtenir des effets nettement supérieurs à un nourrissement plus tardif.

## Revendications

1. Complexe de molybdène de formule I suivante :
A1ₘ-[Mo₂O₂E₂]-Lₙ, I
dans laquelle :- A1 est un cation choisi parmi les cations des métaux alcalins, les cations des métaux alcalino-terreux, les cations des métaux de transition, ou un cation organique PPh₄⁺
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou des larves par l'acarien *Varroa Destructor.*

2. Complexe de molybdène de formule I pour une utilisation selon la revendication 1) **caractérisé en ce que** ledit complexe de molybdène est représenté par la formule I(i) A1ₘ-[Mo₂O₄]-Lₙ ou par la formule I(ii) A1ₘ-[Mo₂O₂S₂]-Lₙ, A1, L, m et n étant tels que définis dans la revendication 1.

3. Complexe de molybdène de formule I pour une utilisation selon la revendication 1 ou 2, **caractérisé en ce que** ledit complexe correspond à la formule II suivante :
A2ₘ-[Mo₂O₂E₂]-Lₙ, II
dans laquelle :
- A2 est un cation choisi parmi les cations des métaux alcalins, les cations des métaux alcalino-terreux, ou les cations des métaux de transition,
- E est choisi parmi O ou S,
- L est un ligand choisi parmi les acides aminés tels que la glycine (gly⁻), la cystéine (L-cys²⁻) ou l'histidine (L-his⁻) ou des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2.

4. Complexe de molybdène de formule I pour une utilisation selon l'une quelconque des revendications 1-3, **caractérisé en ce que** ledit complexe est choisi parmi Na₂[Mo₂O₄(EDTA)].xH₂O (x = 5-6), Li₂[Mo₂O₄(EDTA)].xH₂O (x = 3-6), et (PPh₄)₂[Mo₂O₄(EDTA)].xH₂O (x = 2-5).

5. Complexe de molybdène de formule I pour une utilisation selon l'une quelconque des revendications 1-4, **caractérisé en ce que** ledit complexe est utilisé en une quantité annuelle de 1 à 60 mg/colonie, notamment de 1 à 40 mg/colonie, encore notamment de 1 à 20 mg/colonie, notamment de 1 à 15 mg/ colonie, encore notamment de 1 à 10 mg/colonie, encore notamment de 1,5 à 8 mg/colonie, et plus particulièrement de 2 à 6 mg/colonie.

6. Composition comprenant au moins un complexe de molybdène de formule I suivante :
A1ₘ-[Mo₂O₂E₂]-Lₙ, **I**
dans laquelle :
- A1 est un cation choisi parmi les cations des métaux alcalins, les cations des métaux alcalino-terreux, les cations des métaux de transition, ou un cation organique PPh₄⁺
- E est choisi parmi O ou S,
- L est un ligand choisi parmi les acides aminés tels que la glycine (gly-), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻) le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la prévention de l'infestation des abeilles et/ou des larves par l'acarien *Varroa destructor.*

7. Composition pour une utilisation selon la revendication 6, **caractérisée en ce que** ledit complexe de molybdène correspond à la formule II telle que définie dans la revendication 3.

8. Composition pour une utilisation selon la revendication 6 ou 7, **caractérisée en ce que** ladite composition comprend en outre un ou plusieurs aliments pour les abeilles choisis parmi de l'eau, le pollen, le nectar, le miel, les sucres, les vitamines, les minéraux, les lipides, et les protéines.

9. Complexe tel que défini selon l'une quelconque des revendications 1-5 ou une composition comprenant ledit complexe telle que définie selon les revendications 6-8, pour utilisation dans une méthode de prévention contre l'infestation des abeilles et des larves par l'acarien Varroa Destructor, ladite méthode étant **caractérisée en ce qu'**elle comprend le nourrissement de la colonie avec la quantité globale et annuelle dudit complexe utilisé par colonie étant de 1 à 60 mg/colonie, notamment de 1 à 40 mg/colonie, encore notamment de 1 à 20 mg/colonie, encore notamment de 1 à 15 mg/ colonie, encore notamment de 1 à 10 mg/colonie, encore notamment de 1,5 à 8 mg/colonie, et plus particulièrement de 2 à 6 mg/colonie.

10. Complexe ou composition pour utilisation selon la revendication 9, **caractérisée en ce que** chaque colonie est nourrie par ledit complexe ou ladite composition à une dose unique annuelle de 1 à 60 mg/colonie, notamment de 1 à 40 mg/colonie, encore notamment de 1 à 20 mg, notamment de 1 à 15 mg/ colonie, encore notamment de 1 à 10 mg/colonie, encore notamment de 1,5 à 8 mg, plus particulièrement de 2 à 6 mg.

11. Complément alimentaire pour les abeilles, **caractérisé en ce qu'**il comprend un complexe de molybdène de formule II suivante :
A2ₘ-[Mo₂O₂E₂]-Lₙ, II
dans laquelle :
- A2 est un cation choisi parmi les cations des métaux alcalins, les cations des métaux alcalino-terreux, ou les cations des métaux de transition
- E est choisi parmi O ou S,
- L est un ligand choisi parmi les acides aminés tels que la glycine (gly-), la cystéine (L-cys²⁻) ou l'histidine (L-his⁻) ou des ligands polycarboxylates tels que l'oxalate (Ox²⁻), le citrate (Cit³⁻), l'éthylènediaminetetraacétate (EDTA⁴⁻), le nitrilo-triacétate (HNTA²⁻), et l'imino-diacétate (IDA²⁻)
- m est le nombre de cations et est choisi parmi 0, 1, 2, 3 et 4 ;
- n est le nombre de ligands et est choisi parmi 1 et 2.

12. Complément alimentaire pour les abeilles selon la revendication 11, **caractérisé en ce que** ledit complexe de molybdène est représenté par la formule II(i) A2ₘ-[Mo₂O₄]-Lₙ ou par la formule II(ii) A2ₘ-[Mo₂O₂S₂]-Lₙ, A2, L, m et n étant tels que définis dans la revendication 11.

13. Complément alimentaire pour les abeilles selon la revendication 11 ou 12, **caractérisé en ce que** A2 est un cation choisi parmi Li⁺, Na⁺, K⁺, Ca²⁺, Mg²⁺ et Zn²⁺.

14. Complément alimentaire pour les abeilles selon l'une quelconque des revendications 11 à 13, **caractérisé en ce que** ledit complexe est choisi parmi Na₂[Mo₂O₄(EDTA)].xH₂O (x = 5-6), Li₂[Mo₂O₄(EDTA)].xH₂O (x = 3-6), K₂[Mo₂O₂S₂(LCys)₂].xH₂O, Na₂[Mo₂O₄(LCys)₂].xH₂O, K₂[Mo₂O₂S₂](HNTA)₂].xH₂O, K₂[Mo₂O₂S₂(EDTA)].xH₂O, K₂[Mo₂O₄(HNTA)₂].xH₂O, Mg[Mo₂O₄(EDTA)].xH₂O, Ca[Mo₂O₄(EDTA)].xH₂O, et Zn[Mo₂O₄(EDTA)].xH₂O avec x étant compris entre 3 et 6.

15. Complément alimentaire pour les abeilles selon l'une quelconque des revendications 11 à 14, **caractérisé en ce que** ledit complément alimentaire comprend en outre un ou plusieurs aliments pour les abeilles choisis parmi de l'eau, le pollen, le nectar, le miel, les sucres, les vitamines, les minéraux, les lipides, et les protéines.

16. Complément alimentaire pour les abeilles selon l'une quelconque des revendications 11 à 15, **caractérisé en ce que** ledit complément alimentaire est sous forme de poudre, d'un sirop, d'une pâte ou une solution aqueuse.

17. Complément alimentaire pour les abeilles comprenant un complexe de molybdène de formule II :
A2ₘ-[MO₂O₂E₂]-Lₙ,
dans laquelle :
- A2 est un cation choisi parmi les cations des métaux alcalins, les cations des métaux alcalino-terreux, ou les cations des métaux de transition
- E est choisi parmi O ou S,
- L est un ligand choisi parmi les acides aminés tels que la glycine (gly-), la cystéine (L-cys²⁻) ou l'histidine (L-his⁻) ou des ligands polycarboxylates tels que l'oxalate (Ox²), le citrate (Cit³⁻), l'éthylènediaminetetraacétate (EDTA⁴⁻), le nitrilo-triacétate (HNTA²⁻), et l'imino-diacétate (IDA²⁻),
- m est le nombre de cations et est choisi parmi 0, 1, 2, 3 et 4 ;
- n est le nombre de ligands et est choisi parmi 1 et 2,
pour une utilisation dans la diminution de la mortalité des colonies d'abeilles.

18. Complément alimentaire pour les abeilles pour une utilisation selon la revendication 17, **caractérisée en ce que** ladite utilisation concerne la diminution de la mortalité hivernale des colonies d'abeilles.

19. Complément alimentaire pour les abeilles tel que défini selon l'une quelconque des revendications 11 à 16 pour utilisation dans une méthode pour augmenter la production de miel chez les abeilles, notamment la production de miel à la première récolte.

## Patentansprüche

1. Molybdänkomplex der folgenden Formel I:
A1ₘ-[Mo₂O₂E₂]-Lₙ, I
wobei:- A1 ein Kation ist, ausgewählt aus Alkalimetallkationen, Erdalkalimetallkationen, Übergangsmetallkationen oder einem organischen Kation PPh₄⁺
- E aus O oder S ausgewählt ist,
- L ein Ligand ist, ausgewählt aus Aminosäuren wie Glycin (gly⁻), Cystein (L-cys²⁻) oder Histidin (L-his⁻) oder Polycarboxylatliganden wie Oxalat (Ox²⁻), Citrat (Cit³⁻), Ethylendiamintetraacetat (EDTA⁴⁻), Nitrilotriacetat (HNTA²⁻) und Iminodiacetat (IDA²⁻),
- m die Anzahl der Kationen ist und aus 0, 1, 2, 3 und 4 ausgewählt ist;
- n die Anzahl der Liganden ist und aus 1 und 2 ausgewählt ist,
für eine Verwendung bei der Vorbeugung eines Befalls von Bienen und/oder Larven durch die Milbe *Varroa Destructor.*

2. Molybdänkomplex der Formel I für eine Verwendung nach Anspruch 1),
**dadurch gekennzeichnet, dass** der Molybdänkomplex durch die Formel I(i) A1ₘ-[Mo₂O₄]-Lₙ oder durch die Formel I(ii) A1ₘ-[MO₂O₂S₂]-Lₙ dargestellt wird, wobei A1, L, m und n wie in Anspruch 1 definiert sind.

3. Molybdänkomplex der Formel I für eine Verwendung nach Anspruch 1 oder 2, **dadurch gekennzeichnet, dass der** Komplex der folgenden Formel II entspricht:
A2ₘ-[Mo₂O₂E₂]-Lₙ, II
wobei:
- A2 ein Kation ist, ausgewählt aus Alkalimetallkationen, Erdalkalimetallkationen oder Übergangsmetallkationen,
- E aus O oder S ausgewählt ist,
- L ein Ligand ist, ausgewählt aus Aminosäuren wie Glycin (gly⁻), Cystein (L-cys²⁻) oder Histidin (L-his⁻) oder Polycarboxylatliganden wie Oxalat (Ox²⁻), Citrat (Cit³⁻), Ethylendiamintetraacetat (EDTA⁴⁻), Nitrilotriacetat (HNTA²⁻) und Iminodiacetat (IDA²⁻),
- m die Anzahl der Kationen ist und aus 0, 1, 2, 3 und 4 ausgewählt ist;
- n die Anzahl der Liganden ist und aus 1 und 2 ausgewählt ist.

4. Molybdänkomplex der Formel I für eine Verwendung nach einem der Ansprüche 1 bis 3, **dadurch gekennzeichnet, dass** der Komplex ausgewählt ist aus Na₂[Mo₂O₄(EDTA)].xH₂O (x = 5-6), Li₂[Mo₂O₄(EDTA)].xH₂O (x = 3-6) und (RPh₄)₂[Mo₂O₄(EDTA)].xH₂O (x = 2-5).

5. Molybdänkomplex der Formel I für eine Verwendung nach einem der Ansprüche 1 bis 4, **dadurch gekennzeichnet, dass** der Komplex in einer jährlichen Menge von 1 bis 60 mg/Volk, insbesondere von 1 bis 40 mg/Volk, weiter insbesondere von 1 bis 20 mg/Volk, insbesondere von 1 bis 15 mg/Volk, weiter insbesondere von 1 bis 10 mg/Volk, weiter insbesondere von 1,5 bis 8 mg/Volk und spezieller von 2 bis 6 mg/Volk verwendet wird.

6. Zusammensetzung, umfassend mindestens einen Molybdänkomplex der folgenden Formel I:
A1ₘ-[Mo₂O₂E₂]-Lₙ, I
wobei:
- A1 ein Kation ist, ausgewählt aus Alkalimetallkationen, Erdalkalimetallkationen, Übergangsmetallkationen oder einem organischen Kation PPh₄⁺
- E aus O oder S ausgewählt ist,
- L ein Ligand ist, ausgewählt aus Aminosäuren wie Glycin (gly-), Cystein (L-cys²⁻) oder Histidin (L-his⁻) oder Polycarboxylatliganden wie Oxalat (Ox²⁻), Citrat (Cit³⁻), Ethylendiamintetraacetat (EDTA⁴⁻), Nitrilotriacetat (HNTA²⁻) und Iminodiacetat (IDA²⁻),
- m die Anzahl der Kationen ist und aus 0, 1, 2, 3 und 4 ausgewählt ist;
- n die Anzahl der Liganden ist und aus 1 und 2 ausgewählt ist,
für eine Verwendung bei der Vorbeugung eines Befalls von Bienen und/oder Larven durch die Milbe *Varroa Destructor.*

7. Zusammensetzung für eine Verwendung nach Anspruch 6,
**dadurch gekennzeichnet, dass** der Molybdänkomplex der Formel II wie in Anspruch 3 definiert entspricht.

8. Zusammensetzung für eine Verwendung nach Anspruch 6 oder 7, **dadurch gekennzeichnet, dass** die Zusammensetzung ferner ein oder mehrere Nahrungsmittel für die Bienen umfasst, ausgewählt aus Wasser, Pollen, Nektar, Honig, Zuckern, Vitaminen, Mineralien, Lipiden und Proteinen.

9. Komplex wie nach einem der Ansprüche 1 bis 5 oder definiert oder Zusammensetzung, umfassend den Komplex wie nach den Ansprüchen 6 bis 8 definiert, für die Verwendung in einem Verfahren zum Vorbeugen gegen den Befall von Bienen und Larven durch die Milbe Varroa Destructor, wobei das Verfahren **dadurch gekennzeichnet ist, dass** es das Füttern des Volks mit der Gesamt- und Jahresmenge des Komplexes für das Volk umfasst, wobei die Gesamt- und Jahresmenge des für das Volk verwendeten Komplexes von 1 bis 60 mg/Volk, insbesondere von 1 bis 40 mg/Volk, weiter insbesondere von 1 bis 20 mg/Volk, weiter insbesondere von 1 bis 15 mg/Volk, weiter insbesondere von 1 bis 10 mg/Volk, weiter insbesondere von 1,5 bis 8 mg/Volk und spezieller von 2 bis 6 mg/Volk beträgt.

10. Komplex oder Zusammensetzung für die Verwendung nach Anspruch 9, **dadurch gekennzeichnet, dass** jedes Volk mit dem Komplex oder der Zusammensetzung in einer einzigen jährlichen Dosis von 1 bis 60 mg/Volk, insbesondere von 1 bis 40 mg/Volk, weiter insbesondere von 1 bis 20 mg, insbesondere von 1 bis 15 mg/Volk, weiter insbesondere von 1 bis 10 mg/Volk, weiter insbesondere von 1,5 bis 8 mg, spezieller von 2 bis 6 mg, gefüttert wird.

11. Nahrungsergänzungsmittel für Bienen, **dadurch gekennzeichnet, dass** es einen Molybdänkomplex der folgenden Formel II umfasst:
A2ₘ-[Mo₂O₂E₂]-Lₙ, II
wobei:
- A2 ein Kation ist, ausgewählt aus Alkalimetallkationen, Erdalkalimetallkationen oder Übergangsmetallkationen
- E aus O oder S ausgewählt ist,
- L ein Ligand ist, ausgewählt aus Aminosäuren wie Glycin (gly-), Cystein (L-cys²⁻) oder Histidin (L-his⁻) oder Polycarboxylatliganden wie Oxalat (Ox²⁻), Citrat (Cit³⁻), Ethylendiamintetraacetat (EDTA⁴⁻), Nitrilotriacetat (HNTA²⁻) und Iminodiacetat (IDA²⁻)
- m die Anzahl der Kationen ist und aus 0, 1, 2, 3 und 4 ausgewählt ist;
- n die Anzahl der Liganden ist und aus 1 und 2 ausgewählt ist.

12. Nahrungsergänzungsmittel für Bienen nach Anspruch 11,
**dadurch gekennzeichnet, dass** der Molybdänkomplex durch die Formel II(i) A2ₘ-[Mo₂O₄]-Lₙ oder durch die Formel II(ii) A2ₘ-[Mo₂O₂S₂]-Lₙ dargestellt wird, wobei A2, L, m und n wie in Anspruch 11 definiert sind.

13. Nahrungsergänzungsmittel für Bienen nach Anspruch 11 oder 12, **dadurch gekennzeichnet, dass** A2 ein Kation ist, ausgewählt aus Li⁺, Na⁺, K⁺, Ca²⁺, Mg²⁺ und Zn²⁺.

14. Nahrungsergänzungsmittel für Bienen nach einem der Ansprüche 11 bis 13, **dadurch gekennzeichnet, dass der** Komplex ausgewählt ist aus Na₂[Mo₂O₄(EDTA)].xH₂O (x = 5-6), Li₂[Mo₂O₄(EDTA)].xH₂O (x = 3-6), K₂[Mo₂O₂S₂(LCys)₂].xH₂O, Na₂[Mo₂O₄(LCys)₂].xH₂O, K₂[Mo₂O₂S₂](HNTA)₂].xH₂O, K₂[Mo₂O₂S₂(EDTA)].xH₂O, K₂[Mo₂O₄(HNTA)₂].xH₂O, Mg[Mo₂O₄(EDTA)].xH₂O, Ca[Mo₂O₄(EDTA)].xH₂O und Zn[Mo₂O₄(EDTA)].xH₂O, wobei X zwischen 3 und 6 ist.

15. Nahrungsergänzungsmittel für Bienen nach einem der Ansprüche 11 bis 14, **dadurch gekennzeichnet, dass** das Nahrungsergänzungsmittel ferner ein oder mehrere Nahrungsmittel für Bienen umfasst, ausgewählt aus Wasser, Pollen, Nektar, Honig, Zuckern, Vitaminen, Mineralien, Lipiden und Proteinen.

16. Nahrungsergänzungsmittel für Bienen nach einem der Ansprüche 11 bis 15, **dadurch gekennzeichnet, dass** das Nahrungsergänzungsmittel in Form eines Pulvers, eines Sirups, einer Paste oder einer wässrigen Lösung vorliegt.

17. Nahrungsergänzungsmittel für Bienen, umfassend einen Molybdänkomplex der Formel II:
A2ₘ-[Mo₂O₂E₂]-Lₙ,
wobei:
- A2 ein Kation ist, ausgewählt aus Alkalimetallkationen, Erdalkalimetallkationen oder Übergangsmetallkationen
- E aus O oder S ausgewählt ist,
- L ein Ligand ist, ausgewählt aus Aminosäuren wie Glycin (gly-), Cystein (L-cys²⁻) oder Histidin (L-his⁻) oder Polycarboxylatliganden wie Oxalat (Ox²⁻), Citrat (Cit³⁻), Ethylendiamintetraacetat (EDTA⁴⁻), Nitrilotriacetat (HNTA²⁻) und Iminodiacetat (IDA²⁻),
- m die Anzahl der Kationen ist und aus 0, 1, 2, 3 und 4 ausgewählt ist;
- n die Anzahl der Liganden ist und aus 1 und 2 ausgewählt ist,
für eine Verwendung bei der Reduzierung der Sterblichkeit von Bienenvölkern.

18. Nahrungsergänzungsmittel für Bienen für eine Verwendung nach Anspruch 17, **dadurch gekennzeichnet, dass** die Verwendung die Reduzierung der Wintersterblichkeit von Bienenvölkern betrifft.

19. Nahrungsergänzungsmittel für Bienen, wie nach einem der Ansprüche 11 bis 16 definiert, für die Verwendung in einem Verfahren zum Steigern der Honigproduktion bei Bienen, insbesondere der Honigproduktion bei der ersten Ernte.

## Claims

1. A molybdenum complex of the following formula I:
A1ₘ-[Mo₂O₂E₂]-Lₙ, I
wherein:
- A1 is cation selected from alkali metal cations, alkaline earth metal cations, transition metal cations, or a PPh₄⁺ organic cation
- E is selected from O or S,
- L is a ligand selected from the amino acids such as glycine (gly), cysteine (L-cys²⁻) or histidine (L-his⁻) or polycarboxylate ligands such as oxalate (Ox²⁻), citrate (Cit³⁻), ethylenediaminetetraacetate (EDTA⁴⁻), nitrilotriacetate (HNTA²⁻), and iminodiacetate (IDA²⁻),
- m is the number of cations and is selected from 0, 1, 2, 3 and 4;
- n is the number of ligands and is selected from 1 and 2,
for use in preventing the infestation of bees and/or larvae by the *Varroa Destructor* mite.

2. The formula I molybdenum complex for use according to claim 1, **characterized in that** said molybdenum complex is represented by formula I(i) A1ₘ-[Mo₂O₄]-Lₙ or by formula I(ii) A1ₘ-[Mo₂O₂S₂]-Lₙ, A1, L, m and n being as defined in claim 1.

3. The formula I molybdenum complex for use according to claim 1 or 2, **characterized in that** said complex corresponds to the following formula II:
A2ₘ-[Mo₂O₂E₂]-Lₙ, II
wherein:
- A2 is a cation selected from the alkali metal cations, alkaline earth metal cations, or transition metal cations,
- E is selected from O or S,
- L is a ligand selected from the amino acids such as glycine (gly), cysteine (L-cys²⁻) or histidine (L-his⁻) or polycarboxylate ligands such as oxalate (Ox²⁻), citrate (Cit³⁻), ethylenediaminetetraacetate (EDTA⁴⁻), nitrilotriacetate (HNTA²⁻), and iminodiacetate (IDA²⁻),
- m is the number of cations and is selected from 0, 1, 2, 3 and 4;
- n is the number of ligands and is selected from 1 and 2.

4. The formula I molybdenum complex for use according to any one of claims 1-3, **characterized in that** said complex is selected from Na₂[Mo₂O₄(EDTA)].xH₂O (x = 5-6), Li₂[Mo₂O₄(EDTA)].xH₂O (x = 3-6), and (RPh₄)₂[Mo₂O₄(EDTA)].xH₂O (x = 2-5).

5. The formula I molybdenum complex for use according to any one of claims 1-4, **characterized in that** said complex is used in an annual amount of 1 to 60 mg/colony, especially 1 to 40 mg/colony, even especially 1 to 20 mg/colony, especially 1 to 15 mg/colony, even especially 1 to 10 mg/colony, even especially 1.5 to 8 mg/colony, and more particularly 2 to 6 mg/colony.

6. A composition comprising at least one molybdenum complex of the following formula I:
A1ₘ-[Mo₂O₂E₂]-Lₙ, I
wherein:
- A1 is a cation selected from alkali metal cations, alkaline earth metal cations, transition metal cations, or a PPh₄⁺ organic cation
- E is selected from O or S,
- L is a ligand selected from amino acids such as glycine (gly-), cysteine (L-cys²⁻) or histidine (L-his⁻) or polycarboxylate ligands such as oxalate (Ox²⁻), citrate (Cit³⁻), ethylenediaminetetraacetate (EDTA⁴⁻) nitrilotriacetate (HNTA²⁻), and iminodiacetate (IDA²⁻),
- m is the number of cations and is selected from 0, 1, 2, 3 and 4;
- n is the number of ligands and is selected from 1 and 2,
for use in preventing the infestation of bees and/or larvae by the *Varroa destructor* mite.

7. The composition for use according to claim 6, **characterized in that** said molybdenum complex corresponds to formula II as defined in claim 3.

8. The composition for use according to claim 6 or 7, **characterized in that** said composition further comprises one or more foods for bees selected from water, pollen, nectar, honey, sugars, vitamins, minerals, lipids, and proteins.

9. Complex as defined according to claims 1-5 or composition comprising said complex as defined according to claims 6-8, for use in a method for preventing the infestation of bees and larvae by the Varroa Destructor mite, said method being **characterized in that** it comprises feeding the colony with the overall and annual amount of said complex used per colony being from 1 to 60 mg/colony, especially from 1 to 40 mg/colony, even especially from 1 to 20 mg/colony, even especially from 1 to 15 mg/colony, even especially from 1 to 10 mg/colony, even especially from 1.5 to 8 mg/colony, and more particularly from 2 to 6 mg/colony.

10. The complex or the composition for use according to claim 9, wherein each colony is fed with said complex or said composition at a single annual dose of 1 to 60 mg/colony, especially from 1 to 40 mg/colony, even especially from 1 to 20 mg, especially from 1 to 15 mg/colony, even especially from 1 to 10 mg/colony, even especially from 1.5 to 8 mg, more particularly from 2 to 6 mg.

11. A food supplement for bees, **characterized in that** it comprises a molybdenum complex of the following formula II:
A2ₘ-[Mo₂O₂E₂]-Lₙ, II
wherein:
- A2 is a cation selected from alkali metal cations, alkaline earth metal cations, or transition metal cations
- E is selected from O or S,
- L is a ligand selected from amino acids such as glycine (gly-), cysteine (L-cys²⁻) or histidine (L-his⁻) or polycarboxylate ligands such as oxalate (Ox²⁻), citrate (Cit³⁻), ethylenediaminetetraacetate (EDTA⁴⁻), nitrilotriacetate (HNTA²⁻), and iminodiacetate (IDA²⁻)
- m is the number of cations and is selected from 0, 1, 2, 3 and 4;
- n is the number of ligands and is selected from 1 and 2.

12. The nutritional supplement for bees according to claim 11, **characterized in that** said molybdenum complex is represented by formula II(i) A2ₘ-[Mo₂O₄]-Lₙ or by formula II(ii) A2ₘ-[Mo₂O₂S₂]-Lₙ, A2, L, m and n being as defined in claim 11.

13. The nutritional supplement for bees according to claim 11 or 12, **characterized in that** A2 is a cation selected from Li⁺, Na⁺, K⁺, Ca²⁺, Mg²⁺ and Zn²⁺.

14. The nutritional supplement for bees according to any one of claims 11 to 13, **characterized in that** said complex is selected from Na₂[Mo₂O₄(EDTA)].xH₂O (x = 5-6), Li₂[Mo₂O₄(EDTA)].xH₂O (x = 3-6), K₂[Mo₂O₂S₂(LCys)₂].xH₂O, Na₂[Mo₂O₄(LCys)₂].xH₂O, K₂[Mo₂O₂S₂](HNTA)₂].xH₂O, K₂[Mo₂O₂S₂(EDTA)].xH₂O, K₂[Mo₂O₄(HNTA)₂].xH₂O, Mg[Mo₂O₄(EDTA)].xH₂O, Ca[Mo₂O₄(EDTA)].xH₂O, and Zn[Mo₂O₄(EDTA)].xH₂O with x being between 3 and 6.

15. The nutritional supplement for bees according to any one of claims 11 to 14, **characterized in that** said food supplement further comprises one or several foods for bees selected from water, pollen, nectar, honey, sugars, vitamins, minerals, lipids, and proteins.

16. The nutritional supplement for bees according to any one of claims 11 to 15, **characterized in that** said food supplement is in the form of a powder, syrup, paste or an aqueous solution.

17. A food supplement for bees comprising a formula II molybdenum complex:
A2ₘ-[Mo₂O₂E₂]-Lₙ,
wherein:
- A2 is a cation selected from alkali metal cations, alkaline earth metal cations, or transition metal cations
- E is selected from O or S,
- L is a ligand selected from amino acids such as glycine (gly-), cysteine (L-cys²⁻) or histidine (L-his⁻) or polycarboxylate ligands such as oxalate (Ox²⁻), citrate (Cit³⁻), ethylenediaminetetraacetate (EDTA⁴⁻), nitrilotriacetate (HNTA²⁻), and iminodiacetate (IDA²⁻),
- m is the number of cations and is selected from 0, 1, 2, 3 and 4;
- n is the number of ligands and is selected from 1 and 2,
for use in reducing bee colony mortality.

18. The food supplement for bees for use according to claim 17, **characterized in that** said use relates to reducing the winter mortality of bees.

19. Food supplement for bees as defined according to any one of claims 11 to 16, for use in a method for increasing honey production in bees, especially honey production during the first harvest.
